(19) **Europäisches Patentamt**
**European Patent Office**
**Office européen des brevets**

(11) **EP 2 199 948 A1**

(12) **EUROPEAN PATENT APPLICATION**

(43) Date of publication:
**23.06.2010 Bulletin 2010/25**

(51) Int Cl.:
**G06K 9/00** *(2006.01)* **A61B 5/00** *(2006.01)*
**G06T 7/20** *(2006.01)*

(21) Application number: **08172087.2**

(22) Date of filing: **18.12.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
• **Koninklijke Philips Electronics N.V.**
**5621 BA Eindhoven (NL)**
Designated Contracting States:
**AT BE BG CH CY CZ DK EE ES FI FR GB GR HR HU IE IS IT LI LT LU LV MC MT NL NO PL PT RO SE SI SK TR**
• **Philips Intellectual Property & Standards GmbH**
**20099 Hamburg (DE)**
Designated Contracting States:
**DE**

(72) Inventor: **The designation of the inventor has not yet been filed**

(74) Representative: **Golla-Franz, Anke Lucia**
**Philips**
**Intellectual Property & Standards GmbH**
**Postfach 50 04 42**
**52088 Aachen (DE)**

(54) **Method of plotting a 3D movement in a 1D graph and of comparing two arbitrary 3D movements**

(57) According to the invention, a method for plotting a 3D movement in a 1D graph, is described which comprises the steps of:
a) measuring the orientation values of a 3D motion sensor device for a 3D movement,
b) sub-sampling the orientation values of step a) by using a reduced number of subsample onentation values,
c) assigning a 1D-numerical value to each of the sub-samples of step b),
d) calculating the projection of a sample of the 3D movement on a path defined by the sub-samples by calculating a projection of the sample between each consecutive pair of sub-samples,

e) taking a weighted average over the projections calculated in step d)
f) repeating steps d) and e) for each orientation value of step a), and
g) plotting the 1D numerical values of the samples of the 3D movement in a ID graph.
In this way, an improved and more intuitive method to plot a complex 3D movement in a 1D graph is provided. According to the invention, further a method for comparison of two arbitrary 3D movements and a device for carrying out the method are provided.

**Description**

FIELD OF THE INVENTION

The present invention refers to methods for plotting and comparing a 3D movement in a 1D graph which can be useful in the field of assessing physiotherapy exercises.

BACKGROUND OF THE INVENTION

Recently, research tools are being developed for physiotherapy that enables the patient to execute physiotherapy exercises with less guidance from the therapist. Background for the research is the fact that at the moment physiotherapy is very labour intensive as a physiotherapist can treat only one patient at a time. This makes physiotherapy very expensive, resulting in only a limited number of reimbursed treatments for the patient. The outcome for the patient would be improved if he could do more or longer physiotherapy exercises, but the costs of the physiotherapist being present all the time prevents this.

To reduce the guidance needed by the physiotherapist, computer assisted systems are being developed where the patient wears one or more motion sensors that measure his or her movements. A computer evaluates the movements and on one hand gives real-time feedback to the patient to motivate the patient to perform the exercise in the correct way. On the other hand the computer can generate progress reports for the therapist offline. In this way the therapist can guide for example 5 or 10 patients simultaneously working with this type of system.

In WO 2005/074370 A2 or WO 2008/007292 A1 for instance, devices and methods are being described that comprise recording a template movement and comparison of the actual patient movements sensed by position sensors worn by the patient with the template.

The present technology implies several limitations that relate to the complexity of the rehabilitation apparatuses and the compulsory use of additional optical detection of the movement. Furthermore, the display of a complex 3D movement without the need of optical devices, for instance in a 2D or 1D graph, is difficult to achieve with the presented methods.

Thus, there is a need for the development of a more intuitive and simplified way to provide a method that can plot a complex 3D movement in a 1D graph in order to guide the patient's exercises and give an instant feedback on the quality of the actual movements.

SUMMARY OF THE INVENTION

It would now be desirable to provide an improved and more intuitive method to plot a complex 3D movement in a 1D graph as well as a method for comparison of two arbitrary 3D movements and a device for carrying out the method.

The present invention provides a method for plotting a 3D movement in a 1D graph, comprising the following steps:

    a) measuring the orientation values of a 3D motion sensor device for a 3D movement,

    b) sub-sampling the orientation values of step a) by using a reduced number of sub-sample orientation values,

    c) assigning a 1D-numerical value to each of the sub-samples of step b),

    d) calculating the projection of a sample of the 3D movement on a path defined by the sub-samples by calculating a projection of the sample between each consecutive pair of sub-samples.

    e) taking a weighted average over the projections calculated in step d)

    f) repeating steps d) and e) for each orientation value of step a), and

    g) plotting the 1D numerical values of the samples of the 3D movement in a 1D graph.

In step a) of the inventive method measuring the orientation values of a complex 3D movement by a 3D motion sensor device is carried out. The motion sensor can be worn by a patient on a relevant part on the body, for example on the wrist when doing an exercise for the arm, and the motion sensor measures its orientation in 3D space. The measured orientation can be expressed as a quaternion, but the method can also be used when the orientation is expressed in for example Euler angles or rotation matrices. Useful motion sensors are known in the art and can for example be chosen from the types of inertial sensors like acceleration sensors, rotational sensors, gyro sensors, position sensors, etc.

In step b) of the inventive method sub-sampling the orientation values of step a) by using a reduced number of sub-sample orientation values is carried out to preferably reduce the calculation power needed and smoothen out the 3D movement.

To sub-sample the 3D movement in a reduced number of sub-samples, for example a first sub-sample $Q_0$

equal to the first sample of the respective movement is defined. Next the distance between each sample of the reference movement with $Q_0$ is calculated, using a definition of distance between two orientations as will be described below. The sample which has the largest distance is assumed to be the 'maximum' of the arbitrary movement. In physiotherapy, this maximum can, for example, represent a moment of the respective movement where an arm is fully stretched. Another sub-sample, for instance $Q_4$, equal to this sample is then defined.

Next a sample lying between the samples of $Q_0$ and $Q_4$ that has the same distance to $Q_0$ as to $Q_4$ is sought. This sample can be defined as the second sub-sample $Q_2$. This definition can be repeated for sub-sample $Q_1$ between $Q_0$ and $Q_2$ and sub-sample $Q_3$ between $Q_2$ and $Q_4$. Another repetition of this approach results in finding sub-samples $Q_5$, $Q_6$, and $Q_7$, all lying between $Q_4$ and the end of the reference movement. The number of sub-samples chosen is not critical and even the full number of samples can be chosen to be carried forward to the next step of the inventive method.

One example for a definition of a distance between two orientations can be given as follows for the presumption that the orientation values of the motion sensor are given in quarternions.

The difference quaternion $Q_d$ between two orientations $Q_1$ and $Q_2$ of the motion sensor can be calculated by:

$$Q_d = Q_2 \otimes Q_1^*$$ 
(1)

or:

$$Q_d = Q_1 \otimes Q_2^*$$ 
(2)

where $Q^*$ means the conjugated quaternion of Q and $\otimes$ means the quaternion product.

Note that the interpretation of equation (1) and (2) is slightly different: If $Q_d$ is calculated using (1) the quaternion going from $Q_1$ to $Q_2$ is given, expressed in the coordinate frame $Q_1$. If $Q_d$ using (2) is calculated the quaternion going from $Q_2$ to $Q_1$ is obtained, expressed in coordinate frame $Q_2$. In some applications these subtle differences make a difference, but for the inventive method both equations can be used.

A quaternion that is normalized to the unity length 1 can be interpreted in the following way:

$$Q = \begin{bmatrix} q_w \\ q_x \\ q_y \\ q_z \end{bmatrix} = \begin{bmatrix} \cos\left(\frac{1}{2}\alpha\right) \\ \sin\left(\frac{1}{2}\alpha\right) \begin{bmatrix} x \\ y \\ z \end{bmatrix} \end{bmatrix}$$ 
(3)

For the definition of the distance between two orientations, information about the rotation axis (x, y, z) between the two orientations is not required, but only how much the rotation along this axis is to get from one orientation to the other. In other words, only the first element (the w-element) of the difference quaternion $Q_d$ has to be regarded. This is notated as $\lfloor Q_d \rfloor_w$. As the quaternion Q and -Q represent exactly the same rotation only the absolute value of the first element of Qd, $|\lfloor Q_d \rfloor_w|$ is being of interest.

Thus, the distance $\delta$ between two orientations can be defined as:

$$\delta = 2\arccos\left(\left|\lfloor Q_2 \otimes Q_1^* \rfloor_w\right|\right)$$ 
(4)

which is equal to:

$$\delta = 2\arccos\left(\left|\lfloor Q_1 \otimes Q_2^* \rfloor_w\right|\right)$$ 
(5)

Substituting the definition of the non-commutative quaternion multiplication (A)

$$Q_1 \otimes Q_2 = \begin{bmatrix} w_2 w_1 - [x_2, y_2, z_2] \cdot [x_1, y_1, z_1] \\ [x_2, y_2, z_2] \times [x_1, y_1, z_1] + w_2[x_1, y_1, z_1] + w_1[x_2, y_2, z_2] \end{bmatrix} =$$

$$Q_1 \otimes Q_2 = \begin{bmatrix} w_1 w_2 - x_1 x_2 - y_1 y_2 - z_1 z_2, \\ y_1 z_2 - z_1 y_2 + w_1 x_2 + w_2 x_1, \\ z_1 x_2 - x_1 z_2 + w_1 y_2 + w_2 y_1, \\ x_1 y_2 - y_1 x_2 + w_1 z_2 + w_2 z_1 \end{bmatrix} \quad\quad (A)$$

in formulae (4) and (5) the distance value is obtained as:

$$\delta = 2 \arccos\left(\left| w_1 w_2 + x_1 x_2 + y_1 y_2 + z_1 z_2 \right|\right) \quad\quad (6)$$

which can be interpreted as two times the arccosine of the absolute value of the quaternion inner product of quaternions $Q_1$ and $Q_2$.

Apart from the distance definition as described above under formula (6) also slightly different definitions for the distance between two orientations can be used. For example, but not limited to:

$$\delta = \sqrt{(w_1 - w_2)^2 + (x_1 - x_2)^2 + (y_1 - y_2)^2 + (z_1 - z_2)^2} \quad\quad (7)$$

This definition is less computational demanding but it is less linear for large angles on the other hand.

If a position sensor is used instead of the orientation sensor the notion of angular distance can for this case simply be replaced by the Euclidian distance

$$\left( \delta = \sqrt{(x_1 - x_2)^2 + (y_1 - y_2)^2 + (z_1 - z_2)^2} \right).$$

In step c) of the inventive method a 1D-numerical value is assigned to each of the sub-samples of step b).

To continue the example given above for the choice of - for instance - eight sub-samples, to $Q_0$ a 1D-numerical value of 0.0 is assigned, to $Q_1$ a value of 0.25, to $Q_2$ a value of 0.5, to $Q_3$ a value of 0.75, and to $Q_4$ a value of 1.0. Similarly the 1D-numerical value of 0.75, 05 and 0.25 is assigned to $Q_5$, $Q_6$ and $Q_7$. Alternatively other 1D-numerical values can be assigned. For example such values can be assigned that they represent the cumulative distance along the track: to $Q_0$ a value of 0.0, to $Q_1$ a value equal to the actual distance between $Q_0$ and $Q_1$, to $Q_2$ a value again higher than the value assigned to $Q_1$ by the distance between $Q_1$ and $Q_2$, etcetera.

In step d) of the inventive method a projection between each consecutive pair of sub-sample 1D-numerical values of step c) is being calculated.

For each consecutive pair of sub-samples, for example for $Q_0$ and $Q_1$, $Q_1$ and $Q_2$, and so forth, a triangle is formed with the current orientation estimate Q of the sensor. Like that the triangles Q-$Q_0$-$Q_1$, Q-$Q_1$-$Q_2$, and so forth can be obtained. Then, the lengths of the sides of the aforementioned triangles, namely the distances between the corners of the triangles, are being calculated using the distance definition (6) stated above. Next, the perpendicular projections of the point Q on the line pieces $Q_0 Q_1$, $Q_1 Q_2$, $Q_2 Q_3$ etc. can be calculated. With the assignment of the 1D-numerical values to each of the subsamples a linear interpolation between these numerical values can be carried out to obtain a numerical value for the projections of Q on $Q_0 Q_1$, $Q_1 Q_2$ and so forth.

In step e) , a weighted average over all projections is being taken.

Finally a calculation of the projection of each sample of the 3D movement on the path defined by the sub-samples by repeating steps c) and d) for each orientation value of step a) is carried out. This yields a 1D-number of every sample of the 3D movement which can be plotted in a graph in step f) of the inventive method.

In a further embodiment of the present invention the sub-sample orientation values of step b) can be equidistant in angle.

Generally, sub-samples are chosen to be equidistant in time and not in angle. However, choosing the sub-sample values equidistant in angle and thus non-equidistant in time results in that advantageously a value can be assigned to the amplitude of the movement to each of the sub-samples.

Alternatively, the sub-sample orientation values can be chosen non-equidistant. If the sub-samples are chosen non-equidistant in angle it is advisable to also assign the 1D-numerical values to each sub-sample non-equidistant. By that, a relatively good linear relation between movement and the resulting 1D graph can still be obtained.

In another embodiment of the present invention the orientation values of the 3D movement can be expressed as quaternions.

In another embodiment of the present inventive method step d) can include defining a distance between two orientation values for calculating a projection between the two orientation values and taking a weighted average over all projections.

Although other weighting factors can be used, a weighting w for each of the projections being equal to

$$w = \frac{1}{b + c - a}$$ can advantageously be chosen because it has the advantage that it results in a smooth transition between two line segments. Additionally, it follows closely any orientation estimate Q that is very close to the line between two sub-samples, because then $w$ is large ($b + c - a \approx 0$ in that case). Further, with this projection and weighting it is also possible to extrapolate a movement outside the range of the sub-samples, so a movement that has a larger range than the reference movement is represented in a reasonable way.

A method of the present invention can be advantageously used in physiotherapy, in guiding devices for surgery or aeronautics, or in videogames.

Although the present method is described for being advantageously used in physiotherapy, it can also be used to interpret and plot any 3D path into for example a single number, percentage, graph, colour, intensity, audio pitch, or audio speed.

It is another object of the present invention to provide a method for comparing two arbitrary 3D movements in order to make, for example, physiotherapy less labour intensive and give a tool for assessing the quality of a patient's exercises.

Thus, the present invention also comprises a method of comparing two arbitrary 3D movements, comprising the following steps:

  i) measuring the orientation values of a 3D motion sensor device for a first 3D movement,
  ii) measuring the orientation values of a 3D motion sensor device a second 3D movement, and
  iii) calculating the distance of an orientation value of the second 3D movement from an orientation value of the first movement as a measure for the quality of the second 3D movement compared to the first 3D movement.

In steps i) and ii) of the inventive method measuring the orientation values of a complex 3D movement by a 3D motion sensor device is carried out. The motion sensor can be worn by a patient on a relevant part on the body, for example on the wrist when doing an exercise for the arm, and the motion sensor measures its orientation in 3D space. The measured orientation can be expressed as a quaternion, but the method can also be used when the orientation is expressed in for example Euler angles or rotation matrices.

Useful motion sensors are known in the art and can for example be chosen from the types of inertial sensors like acceleration sensors, rotational sensors, gyro sensors and position sensors, etc.

In step iii) a definition of an angular distance between two orientations is needed. Although not limiting, the definition as described above for the measured orientations expressed as quarternions can be chosen as:

$$\delta = 2 \arccos\left(\left|w_1 w_2 + x_1 x_2 + y_1 y_2 + z_1 z_2\right|\right) \qquad (6)$$

which can be interpreted as two times the arccosine of the absolute value of the quaternion inner product of quaternions $Q_1$ and $Q_2$.

Also, different definitions for the distance between two orientations can be used. For example, but not limited to the following definition can be used:

$$\delta = \sqrt{(w_1 - w_2)^2 + (x_1 - x_2)^2 + (y_1 - y_2)^2 + (z_1 - z_2)^2}$$ (7)

If a position sensor is used, the notion of angular distance can be replaced by the

$$\text{Euclidian distance } \delta = \sqrt{(x_1 - x_2)^2 + (y_1 - y_2)^2 + (z_1 - z_2)^2}.$$

With the definition (6) a correlation function can be defined and calculated for two four dimensional quarternion functions. For example, a correlation number C can be defined as:

$$C = \sum_{i=0}^{N} 2\arccos\left(\left|w_1[i]w_2[i] + x_1[i]x_2[i] + y_1[i]y_2[i] + z_1[i]z_2[i]\right|\right)$$ (8)

where C is the correlation, the functions $w_1[i]$, $x_1[i]$, $y_1[i]$ and $z_1[i]$ are the time discreet functions that represent the quaternion $Q_1$, the functions $w_2[i]$, $x_2[i]$, $y_2[i]$ and $z_2[i]$ are the time discreet functions that represent the quaternion $Q_2$, and N is the number of points that are being compared.

When the number C is small, it means that the accumulated difference between $Q_1$ and $Q_2$ is small, which means that $Q_1$ and $Q_2$ are 'similar'. When C is zero it means that $Q_1$ and $Q_2$ are equal. When C is large, it means that $Q_1$ and $Q_2$ are not similar.

In another embodiment of the inventive method for comparing two arbitrary 3D movements a correlation number C for the similarty of two orientation values is divided by the number of samples N for $Q_1$ and $Q_2$, an average mismatch number between $Q_1$ and $Q_2$ at any particular time (in angles or in radians) is obtained. That means if:

$$corr = N\pi - \sum_{i=0}^{N} 2\arccos\left(\left|w_1(i)w_2(i) + x_1(i)x_2(i) + y_1(i)y_2(i) + z_1(i)z_2(i)\right|\right)$$ (9)

is calculated a correlation that is high (namely $N\pi$) when there is perfect similarity between $Q_1$ and $Q_2$ and a low value when there is low similarity between $Q_1$ and $Q_2$ is got. This is in line with other correlation functions known so that an intuitive measure for the quality of a second 3D movement respective to a first 3D movement can be given.

The quality of a physiotherapy exercise for example can be easily assessed by calculating the quaternion correlation between the actual movement as a second 3D movement and an ideal template or reference movement as a first 3D movement. When the correlation between the actual movement and the motion template is high, the patient performs his exercises correctly. When the correlation is low, the patient performs his exercises not so well.

In another embodiment of the inventive method for comparing two arbitrary 3D movements a time shift can be introduced between the two movements expressed by quaternions and the corresponding correlation function can be defined as:

$$C_{Q_1Q_2}[k] = N\pi - \sum_{i=0}^{N} 2\arccos\left(\left|w_1[i]w_2[k+i] + x_1[i]x_2[k+i] + y_1[i]y_2[k+i] + z_1[i]z_2[k+i]\right|\right)$$ (10)

Equation (10) defines a function and not just one numerical value as in (8) or (9), and each one of the values of the correlation function can be calculated by calculating the correlation between $Q_1[i]$ and a time shifted version of $Q_2[i]$. For C[k], the time shift of $Q_2[i]$ should be k samples.

When using the quaternion correlation the actual movement and the motion template have to be in phase so there is no time delay allowed between the movement of the patient and the motion template, for example. When instead the quaternion correlation function is calculated for any value of k between 0 and the window length $N$, the maximum of this function can be taken. This maximum is an indication for the similarity between the actual, second movement and the motion template as first movement, independent of any delay between the two. Also only portions of the motion template as a first movement can be compared against the actual movement as the second movement. This for example allows individually assessing the beginning, the middle or the end part of the motion.

Further, with the correlation function C[k] defined even an auto-correlation of a quaternion function Q[i] as the quaternion correlation function of Q[i]with itself can be obtained:

$$C_{QQ}[k] = N\pi - \sum_{i=0}^{N} 2\arccos\left(\left|w[i]w[k+i] + x[i]x[k+i] + y[i]y[k+i] + z[i]z[k+i]\right|\right) \quad (11)$$

With the quaternion auto correlation function it is possible for example to check whether a patient is performing a repetitive movement. An algorithm can now take into account the repetition period, the repeatability as well as the motion range and when all these parameters are within certain pre-set boundaries, it outputs that the patient is exercising. The repetition period can be very accurately derived from the position of the first maximum in the quaternion auto correlation function. This information can be extremely helpful for time-stretching purposes. If there is not only a delay between the actual (second) movement and the motion template (first movement), but also a speed difference the repetition period of the actual movement can be measured with the quaternion auto correction function and then time-stretched to match the duration of the motion template. Taking the maximum of the correlation function between the time-stretched actual movement and the motion template gives again an indicator for the performance of the patient.

In another aspect of the present inventive method for comparing two arbitrary 3D movements the second movement can be compared against a set of multiple first movements.

When the actual second movement is not compared to only one motion template as first movement but against multiple motion templates, a gesture recognition can be obtained.

In another embodiment of the inventive method for comparing two arbitrary 3D movements the comparison of the first and second movements are plotted in a 1D graph by carrying out the following steps:

    a) sub-sampling the comparison values of step iii) by using a reduced number of sub-sample values,
    b) assigning a 1D-numerical value to each of the sub-samples,
    c) calculating the projection of a sample of the comparison on a path defined by the sub-samples by calculating a projection of the sample between each consecutive pair of sub-samples,
    d) taking a weighted average over the projections calculated in step c)
    e) repeating steps c) and d) for each comparison value of step iii), and
    f) plotting the 1D numerical values of the samples of the comparison in a 1D graph.

The present invention also comprises a device for plotting and assessing a 3D movement or a comparison between two arbitrary 3D movements, comprising

- a portable 3D motion sensor device for measuring the orientation values of a 3D movement,
- a calculation device for carrying out a method for plotting a 3D movement in a 1D graph, comprising the following steps:

    a) sub-sampling the orientation values of the motion sensor by using a reduced number of sub-sample orientation values,
    b) assigning a 1D-numerical value to each of the sub-samples of step a),
    c) calculating a projection between each consecutive pair of sub-sample 1D-numerical values of step b),
    d) calculating the projection of each sample of the 3D movement on the path defined by the sub-samples by repeating steps b) and c) for each orientation value of the motion sensor, and/or

    - a calculation device for carrying out a method for comparing two arbitrary 3D movements , comprising the following steps:

        i) measuring the orientation values of a 3D motion sensor device for a first 3D movement,
        ii) measuring the orientation values of a 3D motion sensor device a second 3D movement, and
        iii) calculating the distance of an orientation value of the second 3D movement from an orientation value of the first movement as a measure for the quality of the second 3D movement compared to the first 3D movement, and

    - a display device for displaying the plotting of a 1 D graph.

A device of the present invention can be advantageously used in physiotherapy, in guiding devices for surgery or aeronautics, or in videogames.

BRIEF DESCRIPTION OF THE DRAWING

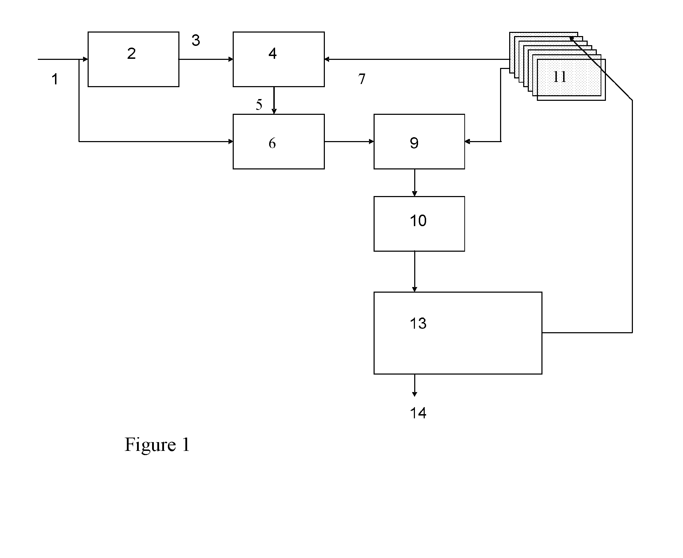

    Fig. 1 schematically shows a diagram for an exemplary method for comparing an actual movement as second movement in comparison with a multitude of template movements as first movement with the goal of gesture recognition.

In the top left box 2 in Figure 1, the quaternion auto correlation function is calculated over the motion sensor signals 1. From this quaternion auto correlation function, the repetition time 3 of the actual movement can be determined.
At the top right boxes 11 of Figure 1 a bank of motion templates is stored, and from this bank one motion template is selected. By dividing the template duration time 7 of the selected template by the repetition time of the actual movement 3, the time stretch rate 4 is calculated.
The motion sensor signals 1 are re-sampled 6 according to this time stretch rate 5, and after that the quaternion correlation function between the time stretched sensor signals and the motion template is calculated 9, of which the maximum is determined 10. This maximum is fed to a loop that scans through all motion templates and finds the motion template with the highest maximum quaternion correlation 13. This is then the recognized gesture 14.
While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

**Claims**

1.    Method for plotting a 3D movement in a 1D graph, comprising the following steps:

        a) measuring the orientation values of a 3D motion sensor device for a 3D movement,
        b) sub-sampling the orientation values of step a) by using a reduced number of sub-sample orientation values,
        c) assigning a 1D-numerical value to each of the sub-samples of step b),
        d) calculating the projection of a sample of the 3D movement on a path defined by the sub-samples by calculating a projection of the sample between each consecutive pair of sub-samples,
        e) taking a weighted average over the projections calculated in step d)
        f) repeating steps d) and e) for each orientation value of step a), and
        g) plotting the 1D numerical values of the samples of the 3D movement in a 1D graph.

2.    The method of claim 1, wherein the sub-sample orientation values are equidistant in angle.

3.    The method of claim 1, wherein the sub-sample orientation values are non-equidistant.

4.    The method of claim 1, wherein the orientation values of the 3D movement are expressed as quaternions.

5.    The method of claim 1, wherein step d) includes defining a distance between two orientation values, using a distance definition for the distance between two orientation values for calculating a projection between the two orientation values.

6.    The method of claim 1, wherein the weighting w for each of the projections in step e) is equal to $w = \dfrac{1}{b + c - a}$ .

7.    A method of comparing two arbitrary 3D movements, comprising the following steps:

i) measuring the orientation values of a 3D motion sensor device for a first 3D movement,

ii) measuring the orientation values of a 3D motion sensor device a second 3D movement, and

iii) calculating the distance of an orientation value of the second 3D movement from an orientation value of the first movement as a measure for the quality of the second 3D movement compared to the first 3D movement.

8. The method of claim 7, wherein a correlation number C can be defined to indicate the mismatch of two orientation values.

9. The method of claim 8, wherein the correlation number C is divided by the number of samples N.

10. The method of claim 9, wherein a time shift can be introduced between the two movements.

11. The method of claim 7, wherein the comparison of the first and second movements are plotted in a 1D graph by carrying out the following steps:

a) sub-sampling the comparison values of step iii) by using a reduced number of sub-sample values,

b) assigning a 1D-numerical value to each of the sub-samples,

c) calculating the projection of a sample of the comparison on a path defined by the sub-samples by calculating a projection of the sample between each consecutive pair of sub-samples,

d) taking a weighted average over the projections calculated in step c)

e) repeating steps c) and d) for each comparison value of step iii), and

f) plotting the 1D numerical values of the samples of the comparison in a 1D graph.

12. Device for plotting and assessing a 3D movement or a comparison between two arbitrary 3D movements, comprising

- a portable 3D motion sensor device for measuring the orientation values of a 3D movement,

- a calculation device for carrying out a method for plotting a 3D movement in a 1D graph, comprising the following steps:

a) sub-sampling the orientation values of the motion sensor by using a reduced number of sub-sample orientation values,

b) assigning a 1D-numerical value to each of the sub-samples of step a),

c) calculating a projection between each consecutive pair of sub-sample 1D-numerical values of step b),

d) calculating the projection of each sample of the 3D movement on the path defined by the sub-samples by repeating steps b) and c) for each orientation value of the motion sensor, and/or

- a calculation device for carrying out a method for comparing two arbitrary 3D movements , comprising the following steps:

i) measuring the orientation values of a 3D motion sensor device for a first 3D movement,

ii) measuring the orientation values of a 3D motion sensor device a second 3D movement, and

iii) calculating the distance of an orientation value of the second 3D movement from an orientation value of the first movement as a measure for the quality of the second 3D movement compared to the first 3D movement, and

- a display device for displaying the plotting of a 1D graph.

13. Use of a method according to claims 1 or 7 in physiotherapy, in guiding devices for surgery or aeronautics, or in videogames.

14. Use of a device according to claim 12 in physiotherapy, in guiding devices for surgery or aeronautics, or in videogames.

Figure 1

Europäisches
Patentamt

European
Patent Office

Office européen
des brevets

# PARTIAL EUROPEAN SEARCH REPORT

**Application Number**

which under Rule 63 of the European Patent Convention EP 08 17 2087
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | MA, YI; SOATTO, STEFANO; KOSECKA, JANA; SASTRY S. SHANKAR: "An invitation to 3-D Vision, Chapter 2" 2004, SPRINGER VERLAG , NEW YORK , XP002529909 ISBN: 978-0-387-00893-6 * whole document, in particular section 2.A "Quaternions and Euler angles for rotations" * * page 15 - page 43 * | 1-6, 11-14 | INV. G06K9/00 A61B5/00 G06T7/20 |
| | ----- | | |
| T | CEDRAS C ET AL: "MOTION-BASED RECOGNITION: A SURVEY" IMAGE AND VISION COMPUTING, vol. 13, no. 2, 1 March 1995 (1995-03-01), pages 129-155, XP001159713 ELSEVIER, GUILDFORD, GB ISSN: 0262-8856 * section "Trajectory Parametrization" * * page 130, right-hand column * | 1-6, 11-14 | |
| | ----- | | |
| | -/-- | | TECHNICAL FIELDS SEARCHED (IPC)  G06K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| The Hague | 29 May 2009 | Neubüser, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

Europäisches
Patentamt

European
Patent Office

Office européen
des brevets

**PARTIAL EUROPEAN SEARCH REPORT**

Application Number

EP 08 17 2087

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | BRIBIESCA E: "A chain code for representing 3D curves" PATTERN RECOGNITION, vol. 33, no. 5, 1 May 2000 (2000-05-01), pages 755-765, XP004243867 ELSEVIER, GB ISSN: 0031-3203 * abstract * | 1-6, 11-14 | |
| A | JONAS A ET AL: "Digital representation schemes for 3D curves" PATTERN RECOGNITION, vol. 30, no. 11, 1 November 1997 (1997-11-01), pages 1803-1816, XP004098481 ELSEVIER, GB ISSN: 0031-3203 * abstract * | 1-6, 11-14 | |
| A | US 2005/288911 A1 (PORIKLI FATIH M [US]) 29 December 2005 (2005-12-29) * paragraphs [0036] - [0058] * | | |
| A | US 6 473 690 B1 (JOSHI RAJASHRI [US]) 29 October 2002 (2002-10-29) * the whole document * | 1-6, 11-14 | |
| A | US 2006/182316 A1 (BANG WON-CHUL [KR] ET AL) 17 August 2006 (2006-08-17) * abstract; figure 6 * | 1-6, 11-14 | |
| A | US 2002/036617 A1 (PRYOR TIMOTHY R [CA]) 28 March 2002 (2002-03-28) * figures 8-27 * | | |
| A | GB 2 434 504 A (KATSIRI ELEFTHERIA [GB]) 25 July 2007 (2007-07-25) * abstract; figures 3,6 * | | |

TECHNICAL FIELDS SEARCHED (IPC)

-/--

EPO FORM 1503 03.82 (P04C10)

Europäisches
Patentamt

European
Patent Office

Office européen
des brevets

PARTIAL EUROPEAN SEARCH REPORT

Application Number

EP 08 17 2087

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| A | GB 2 419 433 A (GLASGOW SCHOOL OF ART [GB]) 26 April 2006 (2006-04-26) * figures 3a,3b,4 * ----- | | |
| | | | **TECHNICAL FIELDS SEARCHED** (IPC) |

Europäisches
Patentamt

European
Patent Office

Office européen
des brevets

**INCOMPLETE SEARCH
SHEET C**

Application Number

EP 08 17 2087

```
Claim(s) searched incompletely:
      1-6,11-14

Reason for the limitation of the search (non-patentable invention(s)):

dsa

      -----

Further limitation of the search

Claim(s) searched incompletely:
      1-6,11-14

Reason for the limitation of the search:

The present application lack clarity, in the claims and descripton, to
such an extent that a complete search could not be performed, Rule 63
EPC. Rather some evidence was collected regarding methods of
representation and comparison of three-dimensional
trajectories/curves/motion.
```

Europäisches
Patentamt

European
Patent Office

Office européen
des brevets

**Application Number**

EP 08 17 2087

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 11, 12 (partially), 13, 14 (partially)

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

**LACK OF UNITY OF INVENTION**
**SHEET B**

Application Number

EP 08 17 2087

The Search Division considers that the present European patent application does not comply with the requirements of unity of invention and relates to several inventions or groups of inventions, namely:

    1. claims: 1-6,11, 12 (partially), 13, 14 (partially)

        Processing three-dimensional movement data involving the calculation of a one-dimensional presentation of the data
           ---

    2. claims: 7-10, 12 (partially), 14 (partially)

        Comparing three-dimensional movements
           ---

## ANNEX TO THE EUROPEAN SEARCH REPORT
## ON EUROPEAN PATENT APPLICATION NO.

EP 08 17 2087

This annex lists the patent family members relating to the patent documents cited in the above-mentioned European search report.
The members are as contained in the European Patent Office EDP file on
The European Patent Office is in no way liable for these particulars which are merely given for the purpose of information.

29-05-2009

| Patent document cited in search report | | Publication date | Patent family member(s) | | Publication date |
|---|---|---|---|---|---|
| US 2005288911 | A1 | 29-12-2005 | NONE | | |
| US 6473690 | B1 | 29-10-2002 | US 6571173 | B1 | 27-05-2003 |
| US 2006182316 | A1 | 17-08-2006 | JP 2006228226 | A | 31-08-2006 |
| | | | KR 20060091589 | A | 21-08-2006 |
| US 2002036617 | A1 | 28-03-2002 | US 2006033713 | A1 | 16-02-2006 |
| GB 2434504 | A | 25-07-2007 | NONE | | |
| GB 2419433 | A | 26-04-2006 | AT 407409 | T | 15-09-2008 |
| | | | EP 1810217 | A1 | 25-07-2007 |
| | | | WO 2006043058 | A1 | 27-04-2006 |
| | | | US 2008192005 | A1 | 14-08-2008 |

EPO FORM P0459

For more details about this annex : see Official Journal of the European Patent Office, No. 12/82

**EP 2 199 948 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005074370 A2
- WO 2008007292 A1